Europäisches Patentamt

European Patent Office

Office européen des brevets

(19)

(11) Publication number: **0 288 299**
**A2**

(12) # EUROPEAN PATENT APPLICATION

(21) Application number: **88303645.1**

(22) Date of filing: **22.04.88**

(51) Int. Cl.⁴: **C 12 Q 1/68**

(30) Priority: **23.04.87 GB 8709652**

(43) Date of publication of application:
**26.10.88 Bulletin 88/43**

(84) Designated Contracting States:
**AT BE CH DE ES FR GB GR IT LI LU NL SE**

(71) Applicant: **ST. MARY'S HOSPITAL MEDICAL SCHOOL**
**Norfolk Place**
**London W2 1PG (GB)**

(72) Inventor: **Williamson, Robert Cystic Fibrosis Genetics**
**Res.Gr**
**Dept.of Biochem. St.Mary's Hospital Medical School**
**University of London London W2 1PG (GB)**

(74) Representative: **Armitage, Ian Michael et al**
**MEWBURN ELLIS & CO. 2/3 Cursitor Street**
**London EC4A 1BQ (GB)**

Claims for the following Contracting State: ES.

(54) **Materials and methods for the diagnosis of cystic fibrosis.**

(57) A region of human genomic DNA from chromosome 7q22-31 has been identified which contains RFLP's in linkage disequilibrium with CF. DNA probes based on fragments incorporating these polymorphisms provide improved CF risk analysis and diagnosis.

Fig.3.

Fig.6.

## Description

## MATERIALS AND METHODS FOR THE DIAGNOSIS OF CYSTIC FIBROSIS

### Field of the invention

This invention relates to DNA hybridisation probes and their use in diagnostic procedures connected with cystic fibrosis.

### Background to the invention

Cystic fibrosis (CF) is a common severe autosomal recessive gene defect affecting the Caucasian population, with a carrier frequency of 1 in 20. In spite of intensive research, the basic defect causing CF remains unknown. Chloride ion impermeability has been demonstrated in both respiratory tract epithelium and sweat gland duct. Tissues from CF patients show a defective beta-adrenergic response, but cAMP production is normal in CF cells, suggesting a defective regulation of chloride channels at the level of an intracellular regulator protein, or the presence of an abnormal regulatory site on the chloride channel itself. We have recently excluded the calmodulin gene, and a catalytic subunit and three distinct regulatory subunits of cAMP-dependent protein kinase, as candidate genes for the CF mutation.

Genetic linkage studies between CF, the protein marker PON, and the DNA markers met, J3.11 (D7S8), 917 (D7S15), TCRB, COL1A2, 7C22 (D7S16) and B79a (D7S13) have localised the CF mutation to chromosome 7q22-31. Data from a collaborative study estimate the recombination fraction between J3.11 and CF to be 0.003, and between met and CF to be 0.004, and suggest that J3.11 and met flank CF. The study shows that the proportion of unlinked (heterogeneous) families is very small; > ;95% of families show linkage to 7q markers.

Both J3.11 and met show significant but mild non-random allelic association (linkage disequilibrium) with CF. A further linkage study with data from CEPH (Centre d'Etude du Polymorphisme Humaine) reference pedigrees and CF families give strong support to the order TCRB/J3.11/CF/met/7C22/79a/COL1A2. The CF mutation is most likely to map between J3.11 and met, but its precise location and identity, either at the level of DNA or at the level of protein expressed by the gene, have not been established, and accordingly diagnostic methods for CF carriers and CF in fetal samples have not been very reliable.

### Summary of the invention

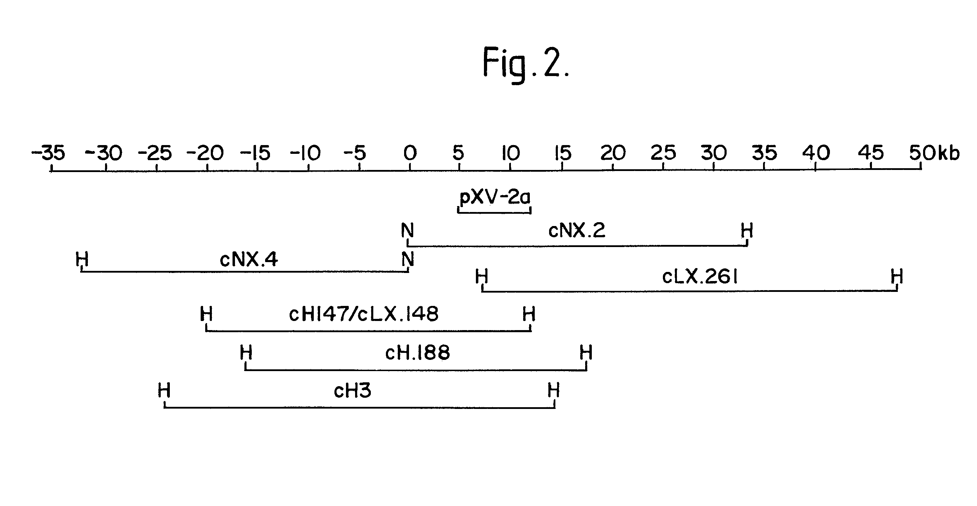

The present invention discloses human genomic DNA possessing polymorphisms in strong linkage disequilibrium with CF. Using linkage disequilibrium, DNA sequences closer to CF than previously known sequences have now been identified. In general terms, the DNA hereof is taken from chromosome 7q22-31, and can be identified as described below in conjunction with the drawings, and in particular Fig. 2, 3 and 6 hereof. The invention in particular provides DNA hybridisation probes corresponding to sequences from the genomic DNA hereof and including one or more of the polymorphisms.

The present invention therefore provides genomic DNA hereof, the DNA probes, methods of using them, and diagnostic kits incorporating them.

As the genetic and physical distance between CF and the probe becomes smaller the degree of allelic association between CF and the polymorphism of the probe would be expected to increase. Disequilibrium studies will be of most value for clones derived from putative coding sequences.

Many vertebrate genes are preceded by a region of DNA rich in the non-methylated dinucleotide CpG, which contain clusters of sites for CpG-methylation sensitive restriction enzymes. These sequences, known as HTF islands ("HpaII tiny fragments"), have a sequence length of between 500-2000 bp, and often include the first exons as well as upstream sequences 5′ to the associated coding gene (Brown, W.R.A. et al. Nature 322, 477-481 (1986) and Bird, A.P. Nature 321, 209-213 (1986)). HTF islands can be used as one set of signals marking for coding sequences.

### Description of the drawings

Figure 1 shows the construction and analysis of a XmaIII/HindIII cosmid library.

Figure 2 shows a schematic representation of the cNX.4-cNX.2 contig.

Figure 3 shows a schematic restriction map of part of the cNX.4-cNX.2 contig, and rare cutting restriction map of the HTF island. Points of cleavage for the restriction enzymes HindIII (H), PstI (P), SacII (S), NotI (N), BamHI (B) and EcoRI (E) are indicated. Cloned DNA segments from human recombinant cosmids are indicated on the top.

Figures 4 and 5 show the segregation of the allelic systems detected for pXV.2c and pCS.7. In Fig. 4, pXV-2c reveals a diallelic polymorphism (A1, 2.1 kb; A2, 1.4 kb) and a constant band of 1.2 kb with TaqI. In Figure 5, pCS.7 detects a two allele polymorphism (A1, 0.67 kb, A2, 0.47 kb) and a constant band of 0.22 kb with HhaI (a methylation-sensitive restriction enzyme). Squares, male; circles, female; solid symbols, CF individuals. DNA was isolated from lymphocytes of family members, 5g of DNA were digested with TaqI or HhaI, electrophoresed and transferred to nylon filters.

Figure 6 shows the location of four RFLPs closely linked to the HTF island detected by the probe pCS.7. The extent of each cosmid clone is shown above the DNA strand, (13). DNA polymorphisms are indicated by an arrow; respective probes are in brackets. HTF indicates the HTF island.

Figure 7 shows segregation of the allelic system detected for pKM.19 in a family with one affected CF child. pKM.19 reveals a diallelic polymorphism (7.6 and 6.8 kb) in Pstl restriction digests. 5µg of DNA were digested with Pstl, electrophoresed in a 0,8% agarose gel and hybridized with pKM.19.

Figure 8 shows a pattern of DNA polymorphic fragments detected with pPT-3 for the enzyme Banll in a family with two affected CF sibs. DNA electrophoresis was in 1.2% agarose. pPT-3 detects a diallelic polymorphism in Banll digests (0.7 and 0.5 kb).

Figure 9 shows the scheme for PCR directed amplification of pCS.7.

## Detailed description of the invention

### A. Detection of a HTF island in the CF region

Several mouse/human hybrid cell lines were constructed that contain transgenomes including the markers most closely linked to CF using chromosome mediated gene transfer (CMGT) (Scambler, P.J. et al. Nucleic Acids Res. 14, 7159-7174 (1986)). One of these cell lines, Elll, contained approximately 10 megabases (Mb) of human DNA, largely from chromosome seven. Line Cll contained approximately 4 Mb of human DNA. Both of these lines, which contain the markers J3.11, met and B79a, proved useful for the physical location of new markers to this region.

To select regions of the genome which identify coding sequences, a selection technique was used to isolate clones derived from non-methylated CpG-rich regions of the transgenomes. The procedure involved the construction of a cosmid library using a rare cutting restriction enzyme sensitive to methylation ("non-methylated CpG rare cutter cosmid library").

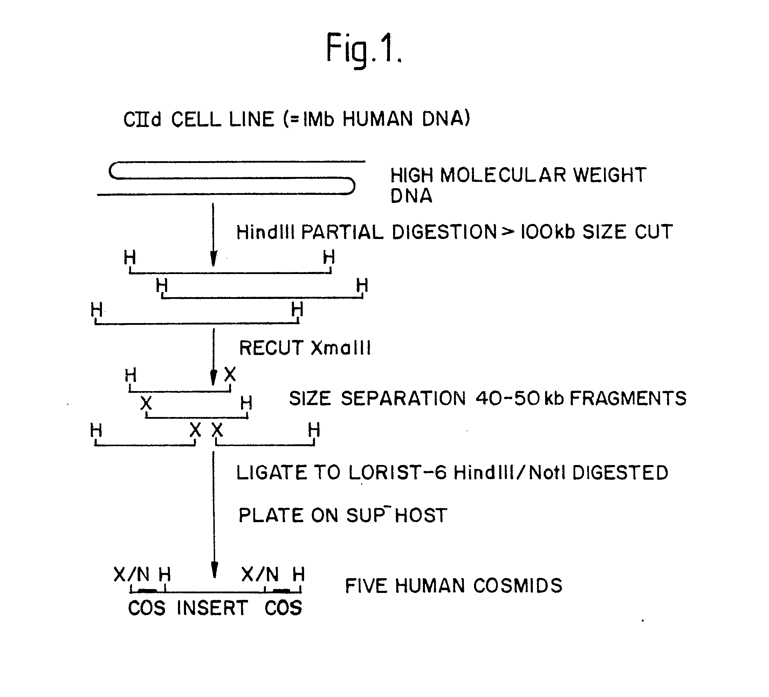

The library was constructed using the method outlined in Fig. 1 from cell line Clld, which is a Cll subclone that has lost some human chromosome seven sequences including J3.11, and contains about 1 Mb of human DNA. High molecular weight DNA from the cell line Clld was partially digested with Hindlll and recut with Xmalll. The Xmalll (Eagl) restriction enzyme was chosen because it cuts relatively infrequently and is methylation-sensitive (McClelland, M. &; Nelson, M. Nucleic Acids Res. 13, 201-207 (1985)). Therefore the construction of a cosmid library from a transgenome 1 Mb in length, using Xmalll as cloning site, allows the identification both of set of space "milestones" at intervals of several hundred kilobases (kb) along this region of chromosome seven, and of putative coding sequences by the identification of HTF islands. The 40-45 kb fraction was isolated and ligated to the Notl/Hindlll sites of cosmid vector Lorist-6, a modification of Lorist-B (Cross, S.H. &; Little, P.F.R. Gene 49, 9-22 (1986)) and packaged in vitro (Grosveld, F.G. et al. Gene 13, 227-237 (1982)) using Gigapack (Stratagene).

The cosmid library constructed contained $10^5$ recombinants. Five different human cosmid clones, designated cNX.1-cNX.5, were identified using two rounds of screening by hybridisation with labelled placental human DNA as the probe. DNA from each cosmid was isolated and digested with Xmalll/Hindlll and Notl/Hindlll, and analysed (Estivill, X. &; Williamson, R. Nucleic Acids Res. 15, 1415-1425 (1987)). The average insert size was 40 kb. Single copy DNA fragments were subcloned into the plasmid vector pUC13 and into the cosmid vector Lorist-6. Subclones of single copy fragments were then used individually as hybridisation probes. Four of the five clones were assigned to chromosome seven using a panel that contains DNA from cell line clone 21 (C1121) which contains the entire human chromosome seven in a mouse background (Croce, C.M. &; Kaprowoski, H. J. Exp. Med. 139, 1350-1353 (1974)), clone 36IS (C136) 7p1.3-7qter as its only human component (Knowles, B.B. et al. J. Exp. Med. 145, 314-326 (1977)) Cll DNA, and total human and mouse DNAs.

A 7 kb Hindlll single copy fragment (designated pXV-2a) from cosmid cNX.2 hybridised to a 7 kb fragment in Hindlll digests of total human DNA and DNA from C121, C136 and Cll, and to 2.8 kb and 2.3 kb fragments of mouse DNA, C121, C135 and Cll. This demonstrated that pXV-2a is located at human chromosome 7q22-7q311 and that the Clld cell line does not contain gross rearrangements or deletions at this site.

pXV-2a was used to screen a cosmid bank of 550 human clones from the cell line Elll (Estivill et al supra). Many of the cosmid clones were analysed, ordered into contiguous overlapping maps ("contigs") (Coulson, A. et al. Proc. Natl. Acad. Sci. U.S.A. 83, 7821-7825 (1986)), and the contigs in turn studied using pulse field gradient electrophoresis (PFGE) gels (Smith, C.L. et al. Genet. Eng. 8, 45-70 (1986)) and rare cutter restriction mapping (Estivill et al supra). Eight overlapping cosmids were detected (Fig. 2). A second clone from the Xmalll/Hindlll cosmid library, cNX.4, was found to overlap with cosmids from the same contig, but not with cNX.2. The cosmid contig including the "linked" Xmalll cosmids cNX.4-cNX.2 extends over a region of about 80 kb (Fig. 2). We fingerprinted these cosmids using a modification of the approach of Coulson et al (supra), and proved both the overlap and map order. The isolated contig did not overlap with another contig 160 kb long, including met, which we had characterised, giving a minimum distance of 120 kb between pXV-2a and met.

To identify human cosmids containing rare cutter restriction sites, miniprepped cosmid DNAs from a cosmid bank (Estivill et al supra) were digested with Hindlll or Hindlll/Sall, the fragments were analysed by agarose gel electrophoresis and transferred to nylon membranes. Notl (eight bases long) and BssHll (seven bases long) oligonucleotides were used as probes. Hybridisation conditions for Notl have been described (Estivill et al supra). For BssHll hybridisation and washing were carried out at 24°C.

cNX.2 and cNX.4 both contained a Notl site, which was consistent with the cloning procedure as the Xmalll site includes the internal six base pairs of the eight-long Notl recognition sequence. cLX.148, a cosmid containing both a Notl and a BssHll site (detected using Notl and BssHll oligonucleotides) overlapped with

both XmaIII cosmids (Fig. 2) and confirms that cNX.2 and cNX.4 each contained one half of a single NotI site, and were adjacent to one another.

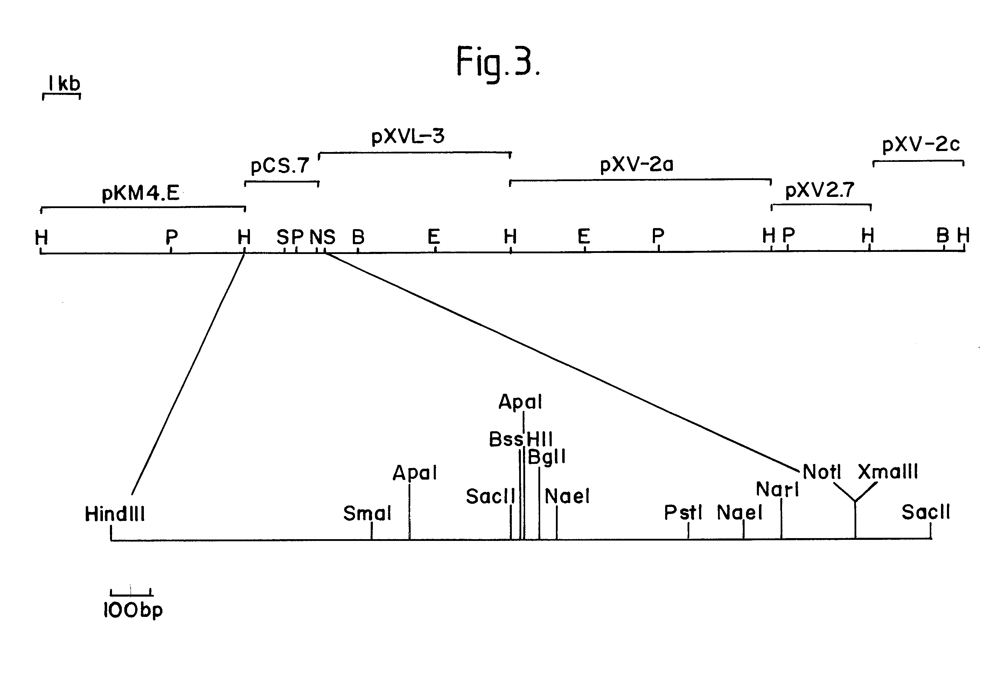

A rare cutter restriction map of cosmids cLX.148/cH.147, containing the NotI site, using both oligonucleotide probes and restriction digests for several rare cutters, was constructed (Fig. 3). A fragment of 1500 bp contained eleven restriction sites for eight such enzymes. This cluster of sites, and the sensitivity of the genomic DNA to XmaIII (proved in the construction of the cosmid library), suggested a high frequency of the non-methylated CpG dinucleotide in this region of the genome; the main criterion for a HTF island.

To define more precisely a non-methylated clustered area for the enzyme HpaII (which is methylation-sensitive and defines an HTF island) genomic DNA from several individuals was digested with SacI, SacI/HpaII and SacI/MspI. The DNA was hybridised with a 2.0 kb NotI/HindIII fragment, derived from cosmid cNX.4, containing the putative "island" (designated pCS.7). Several fragments smaller than 300 bp were obtained for both HpaII and MspI. The pattern obtained using pXV.2a against the same blot showed only one HpaII non-methylated site in a total length of more than 20 kb of genomic DNA which this 7 kb fragment hybridised to.

To map the location of the HTF island, and particularly its relation to the met locus, we used two subclones of the cNX.4-cNX-2 contig (pCS.7 and pXV-2c), which flank the HTF NotI site, to probe Southern blots of NotI-digested human DNA separated using PFGE. pmetD recognised a simple two allele restriction fragment length polymorphism (RFLP) with NotI on PFGE, with bands at 820 and 470 kb. In patients with CF the less frequent allele was found in 2 of 10 individuals (allele frequence 10%).

pXV-2c is polymorphic for the same NotI site as met, and hybridises to the same 820 kb fragment, but in this case the less frequent allele generates a fragment of 350 kb rather than 470 kb. The polymorphic NotI site is therefore within the 820 kb NotI fragment and is flanked by the probes pmetD and pXV-2c. The NotI site within the cNX.4-cNX.2 contig, of which pXV-2c is a part, must be situated at one end of this 820 kb fragment, as this fragment is also recognised by pmetD. The met locus has been reported as being within 150 kb of the NotI site at the other end of the 820 kb fragment (Michiels, F. et. al. Cell (submitted); therefore XV-2c and metD must be separated by at least 650 kb. XV-2c is about 15 kb away from the HTF island. When pCS.7 (which maps at the other half of the NotI site of the cNX4-cNX.2 contig) was hybridised to the same blot a NotI fragment larger than 1 Mb was observed. From these data we can infer that we have jumped 650-750 kb from the met locus.

**B. A human genomic sequence in disequilibrium with the CF locus**

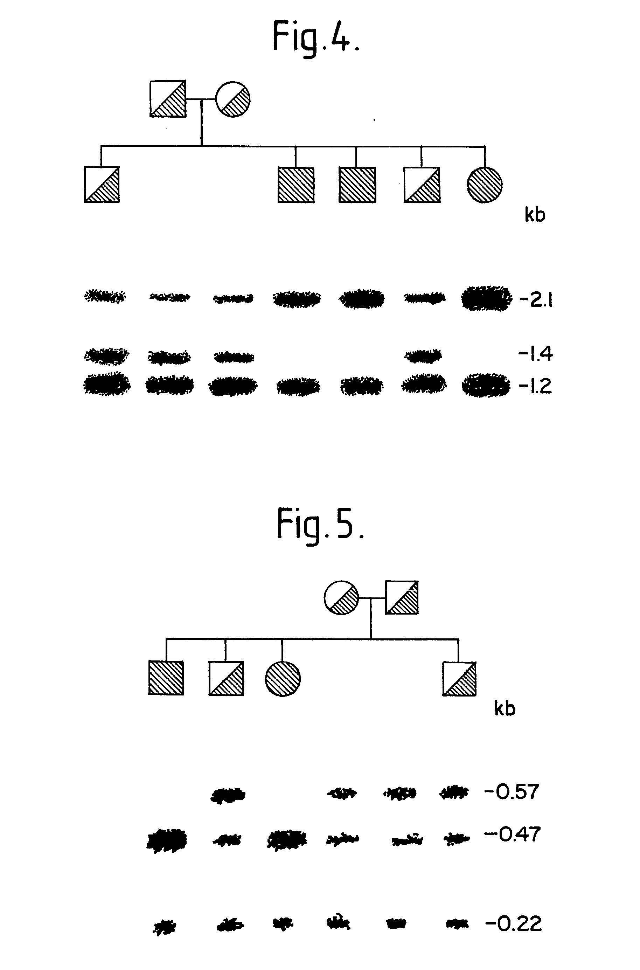

Two probes, pXV-2C and pCS.7, derived from the cosmid contig were found to detect frequent RFLPs. In order to assess the genetic and physical proximity of XV-2c and CS.7 to CF, these markers were used for segregation analysis in both our reference CF families and in families ascertained by showing recombination between CF and the most closely linked markers, and for studies of linkage disequilibrium. The results as shown in Tables 1 and 2 and in Figs. 4 and 5.

**Table 2**

**XV-2c/CS.7 Haplotype distribution of the CF alleles**

| Haplotype | pXV-2c Taq1 | pCS.7 HhaI | Normal alleles | (%) | CF alleles | (%) |
|---|---|---|---|---|---|---|
| A | 1 | 1 | 9 | (14) | 0 | (0) |
| B | 1 | 2 | 22 | (34) | 66 | (94) |
| C | 2 | 1 | 28 | (43) | 0 | (0) |
| D | 2 | 2 | 6 | (9) | 4 | (6) |
| Total | | | 65 | (100) | 70 | (100) |

Alleles are defined in Table 1.

pXV-2c detects a RFLP (A1 0.47, A2 0.53) in TaqI digests (Fig. 4). pCS.7 detects a RFLP (A1 0.56, A2 0.44) in HhaI digests (Fig. 5). No recombination with CF was observed either with pXV-2c or with pCS.7 in six informative families showing a crossover between CF and J3.11, or 7C22 and/or B79a. Two met recombinant families were not informative in the relevant parent for either pCS.7 or pXV-2c.

Combining the cross-over data presented here with the data for probe-probe genetic order (Lathrop, G.M. et al. Science (submitted)), the PFGE results given above, and the data on met activation in the transgenomic cell lines (Scambler et al supra and Park, M. et al. Cell 45, 895-904 (1986)), then the order of loci is:

7qter-J3.11-(CF/CS.7/XV-2c)-met-7C22-B79a-7cen

In 195 out of 213 chromosomes studied the CF mutation is associated with the pXV-2c Al allele (2.1 kb), and in 70 out of 71 chromosomes with the pCS.7 A2 allele (0.47 kb) (Table 1). This suggests that CF is closer to CS.7 than to XV-2c.

The only CF family studied in which the CF chromosomes is associated with pCS.7 allele A1 is also the only "cross-over family" (KB) in which XV-2c and met and 7C22) recombines with CF. This family also shows a second apparent recombination event at the CF locus in a previous generation. We do not know whether sampling or laboratory error, multiple recombinations, or heterogeneity explains this exceptional family. Further analysis and evaluation of the data is given in the paper derived from this work: Estivill et al, Nature, (1987), Vol. 326, No. 6116, pp. 840-845.

The data important implications for prenatal diagnosis and for carrier exclusion (as against carrier detection) in the general population. Almost 95% of the carriers for CF studied so far (140 persons in all) contains at least one chromosome with haplotype B (1,2). This haplotype (the CF haplotype in 94% of chromosomes) occurs in only 34% of the normal population (Table 2). Therefore, individuals with haplotypes A and C, whether homozygous or heterozygous, have a considerably reduced risk of being carriers for CF as compared to the 1 in 20 average risk in the British population; this includes > ;55% of the normal chromosomes. On the other hand, a homozygote for the haplotype B has approximately a 1/7 risk of being carrier for CF. The association of CF to pCS.7 allele 2 may, after confirmation in a larger population sample, allow accurate carrier exclusion on its own.

## C. Isolation of two further probes and further analysis with all four probes

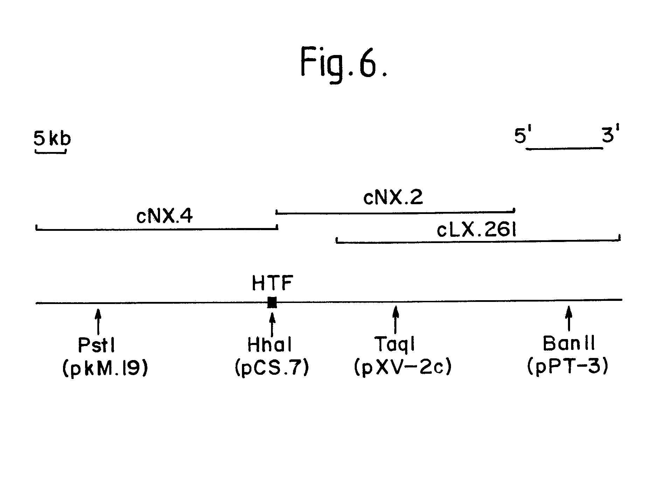

Two further DNA probes, pKM.19 and pPT-3, were isolated from the contigs as shown in Fig. 6, and the disequilibrium was analysed between all four markers and CF for 178 CF and 175 normal chromosomes from four different European populations.

The four probes map relative to the HTF island as shown in Figure 6. Probe pXV-2c is as described above. Probe pCS.7-92, a subclone of pCS.7 (Estivill et al., 1987), is a 1.6 kb HindIII/PstI insert subcloned in pUC13 which detects the same HhaI polymorphism as pCS.7. pKM.19 is a 1 kb EcoRI fragment derived from cosmid cNX.4 and subcloned into pUC13. pPT-3 is a 1.1 kb EcoRI fragment, derived from a cDNA clone of the gene transcribed downstream from the HTF island and subcloned in pSP6.

The four RFLPs analysed were detected with PstI (pKM.19; 7.8/6.6 kb), HhaI (pCS.7-92; 0.6/0.4 kb), TaqI (pXV-2c; 2.1/1.4 lb), and BanII (pPT-3; 0.6/0.5 kb). The polymorphisms detected by pXV-2c and pCS.7-92 are described above.

Analysis of DNA haplotypes in British, Danish, Spanish and Finnish populations suggests that a single mutational event accounts for approximately 85% of cases of CF. Until the mutation(s) causing CF have been completely defined, haplotype analysis with these markers permits accurate prenatal diagnosis, and improves indirect carrier detection/exclusion for each population.

DNA samples were obtained from a total of 89 European families (30 British, 20 Danish, 20 Spanish and 19 Finnish). The families were ascertained through cystic fibrosis clinics; diagnosis was based on clinical features and sweat test. DNA samples were obtained from both parents and at least one affected child; in three cases, paternal DNA was unavailable. None of the parental matings were known to be consanguinous, no non-paternity was found, and all the families were white Caucasian. 39 of the families, each with more than one affected child, had been previously analysed with the markers J3.11 and met and showed no recombination with either marker.

The genotype of each individual was scored for the two alleles, using the code "-" for the longer fragment and " + " for the shorter fragment, corresponding to the absence or presence of the restriction site under test. Haplotypes for both parental chromosomes (normal and CF allele) were established by hand using the CF child in each family to establish linkage phase; the possibility of recombination within the small genetic interval studied is disregarded in this analysis. In rare cases where it was not possible to establish phase, the genotype of the individual at that locus was discarded.

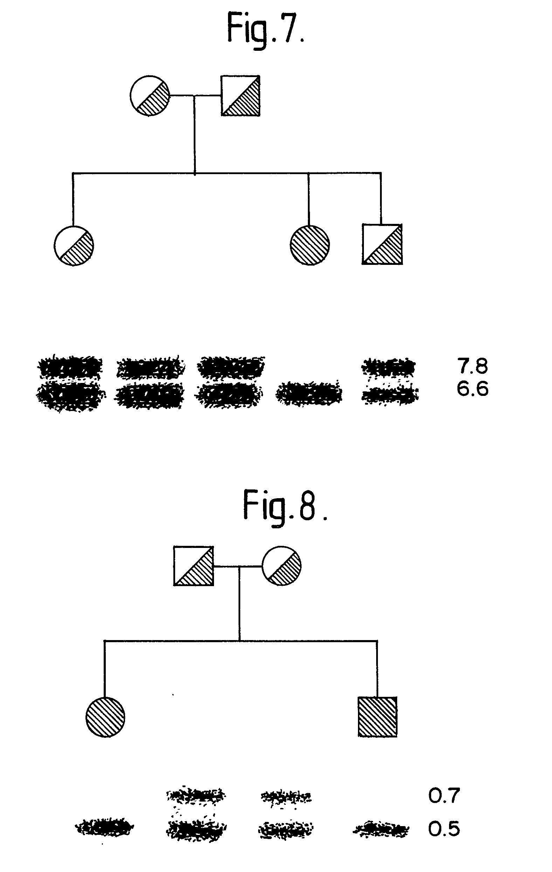

Figures 7 and 8 show the segregation pattens of PstI and the BanII polymorphisms respectively in two CF pedigrees.

## D. Haplotype associations with CF

Distributions of alleles for each polymorphism are shown for CF and normal chromosomes in British, Danish, Spanish and Finnish populations (Table 3). Similar allele frequencies are found for normal chromosomes in all the populations. Tables 3 and 4 list the results of contingency table tests for disequilibrium between CF and each marker. Markedly significant disequilibrium (p ≪ 0.001) is found between CF and KM.19, CS.7 and XV-2c. There is significant variation in the strength of disequilibrium between CF and all four markers in the four populations under study (Table 4). In this sample KM.19 and CS.7 both show stronger linkage disequilibrium with CF than XV-2c, although the confidence limits at mean odds ratio $\pm$ 1.96 SEM overlap. However, it is the distribution of the alleles on normal chromosomes, which varies for the three markers, which contributes to a higher disequilibrium for KM.19 and CS.7 relative to XV-2c. Although for pooled chromosomes there is no overall evidence of disequilibrium between CF and PT-3, the Spanish population singly shows a significant association.

# TABLE 3

## Distribution of Alleles for the *D7S23* Locus in European Populations

| Locus and alleles | Population | Alleles | | | | Relative risk | Standardized disequilibrium coefficient |
| | | CF | | Normal | | | |
| | | − | + | − | + | | |
|---|---|---|---|---|---|---|---|
| pKM.19/*Pst*I | British | 6 | 54 | 44 | 16 | 0.04 | −0.64 |
| | Danish | 0 | 40 | 29 | 10 | 0.004 | −0.77 |
| | Spanish | 8 | 32 | 25 | 14 | 0.15 | −0.45 |
| | Finnish | 7 | 31 | 27 | 10 | 0.09 | −0.55 |
| | Total· | 21 | 157 | 125 | 50 | 0.05 | −0.61 |
| pCS.7/*Hha*I | British | 5 | 55 | 43 | 17 | 0.039 | −0.65 |
| | Danish | 0 | 40 | 27 | 12 | 0.006 | −0.65 |
| | Spanish | 8 | 32 | 24 | 15 | 0.16 | −0.42 |
| | Finnish | 6 | 32 | 22 | 15 | 0.138 | −0.45 |
| | Total | 19 | 159 | 116 | 59 | 0.06 | −0.57 |
| pXV-2c/*Taq*I | British | 53 | 7 | 29 | 31 | 7.62 | +0.43 |
| | Danish | 39 | 1 | 15 | 24 | 41.60 | +0.63 |
| | Spanish | 32 | 8 | 21 | 18 | 3.30 | +0.28 |
| | Finnish | 35 | 3 | 16 | 21 | 13.22 | +0.52 |
| | Total | 159 | 19 | 81 | 94 | 9.49 | +0.46 |
| pPT-3/*Ban*II | British | 16 | 36 | 11 | 41 | 1.63 | +0.11 |
| | Danish | 16 | 24 | 13 | 25 | 1.27 | +0.06 |
| | Spanish | 3 | 37 | 14 | 25 | 0.16 | −0.35 |
| | Finnish· | 13 | 25 | 10 | 25 | 1.29 | +0.06 |
| | Total | 43 | 122 | 48 | 116 | 0.85 | −0.04 |

*Note.* Fragment sizes of alleles are described in the text. CF, cystic fibrosis; +, presence of a given restriction enzyme site; −, absence of the restriction enzyme site.

0 288 299

TABLE 4

χ² Values for Heterogeneity Tests of Linkage Disequilibrium between CF and Markers, Overall Significance of Disequilibrium, Mean Relative Risks, and Associated Standard Errors

| Test | Probe/RFLP | | | |
|------|-----------|------|------|------|
| | pKM19/*Pst*I | pCS.7/*Hha*I | pXV-2c/*Taq*I | pPT-3/*Ban*II |
| Variation of disequilibrium by population (3 *df*) | 10.1** | 11.3** | 8.3* | 10.3** |
| Disequilibrium with CF (1 *df*)[a] | 90.8*** | 79.2*** | 57.0*** | 0.0 |
| Mean relative risk | 0.07 | 0.08 | 8.0 | 1.0 |
| Standard error of mean relative risk | 0.02 | 0.02 | 2.2 | 0.25 |

[a] Taking account of variation between populations. *df*, degrees of freedom.

  * $P < 0.05$.

  ** $P < 0.025$.

  *** $P < 0.001$.

0 288 299

The distribution of haplotypes for the four sites, for both normal and CF chromosomes, in the four groups is presented in Table 5. Eight haplotypes out of the 16 possible haplotypes account for 94% of normal chromosomes and just three haplotypes are found on 91% of CF chromosomes. 85% of the CF chromosomes are of haplotypes 1, + + - +, and 2, + + --; these haplotypes account for only 16% of the normal chromosomes. The next largest 'family' of CF haplotypes are 3, - - + +, and 4, - - + - (7%); these two haplotypes are found on 43% of normal chromosomes. Haplotypes 3 and 4 cannot be derived from haplotype 1 or 2 by a single point mutation or crossover event. 8% of CF chromosomes do not belong to these common haplotypes and may be derived from them either by point mutation or recombination.

## TABLE 5

### Haplotypes for the *D7S23* Locus in Four European Populations

| | Haplotype | | | | British | | Danish | | Spanish | | Finnish | | Total | |
|---|---|---|---|---|---|---|---|---|---|---|---|---|---|---|
| | *Pst*I | *Hha*I | *Taq*I | *Ban*II | CF | N | CF | N | CF | N | CF | N | CF (%) | N (%) |
| 1 | + | + | − | + | 33 | 5 | 23 | 1 | 27 | 4 | 17 | 3 | 100 (57) | 13 (8) |
| 2 | + | + | − | − | 18 | 3 | 16 | 2 | 2 | 3 | 12 | 4 | 49 (28) | 12 (8) |
| 3 | − | − | + | + | 4 | 22 | —— | 10 | 5 | 10 | 2 | 14 | 11 (6) | 57 (36) |
| 4 | − | − | + | − | 1 | 2 | —— | 5 | —— | 2 | —— | 2 | 1 (.6) | 11 (7) |
| 5 | + | + | + | + | 1 | 3 | 1 | 4 | 2 | 4 | —— | 1 | 5 (3) | 13 (8) |
| 6 | − | + | − | + | 1 | 3 | —— | 3 | —— | 2 | —— | 2 | 1 (.6) | 10 (6) |
| 7 | − | − | − | + | —— | 8 | —— | 3 | 3 | 5 | 4 | 2 | 7 (4) | 18 (11) |
| 8 | − | − | − | − | —— | 4 | —— | 3 | —— | 7 | —— | 2 | —— | 16 (10) |
| 9 | + | − | − | − | —— | 1 | —— | —— | —— | —— | —— | —— | —— | 1 (.6) |
| 10 | + | + | + | − | —— | 1 | —— | —— | ·1 | 2 | —— | —— | 1 (.6) | 3 (2) |
| 11 | − | + | + | − | —— | —— | —— | —— | —— | —— | —— | —— | —— | —— |
| 12 | − | + | − | − | —— | —— | —— | —— | —— | —— | 1 | 2 | 1 (.6) | 2 (1) |
| 13 | + | − | − | + | —— | —— | —— | —— | —— | —— | —— | —— | —— | —— |
| 14 | + | − | + | + | —— | —— | —— | 1 | —— | —— | —— | —— | —— | 1 (.6) |
| 15 | − | + | + | + | —— | —— | —— | 1 | —— | —— | —— | 1 | —— | 2 (1) |
| 16 | + | − | + | + | —— | —— | —— | —— | —— | —— | —— | —— | —— | —— |
| | | Total | | | 58 | 52 | 40 | 33 | 40 | 39 | 36 | 33 | 176 (100) | 159 (100) |

*Note.* CF, cycstic fibrosis; N, normal.

Alleles at the three RFLP sites marked by pKM.19, pCS.7 and pXV-2c are in significant disequilibrium with CF in all four populations studied, and in the Spanish group a fourth RFLP also shows association with the disease. This raises the possibility that an independent mutation occurred which has been inherited in some of the Spanish families, and is marked by the PT-3/BanII polymorphism.

The variation in haplotype frequencies between the normal and CF chromosomes in the four populations studied offers clues as to the sites and numbers of mutations causing the disease. In the Danish population just two haplotypes account for nearly all the CF chromosomes, which differ from one another at a single site, both are uncommon on normal chromosomes. This profound disequilibrium with CF in the Danish population is exceptional for most hereditary diseases studied so far, and argues strongly that a single molecular mutation accounts for the great majority of cases of cystic fibrosis in the Danish population. A similar haplotype distribution was found in the other European populations studied; the same two haplotypes [1 + 2] account for 85% of the CF chromosomes.

A third haplotype [3] which differs at the three polymorphic sites in highest disequilibrium is found at a frequency of 7%. This suggests that a second and independent mutational event causing cystic fibrosis has occurred on another chromosome with a different haplotype.

The pooled data for all populations studied shows highest levels of association at the 5' end and upstream of the coding sequence, where a "plateau" of disequilibrium occurs.

The markers are highly informative; because of the disequilibrium a higher proportion of CF families are informative for KM.19 and XV-2c than would be predicted on RFLP frequency alone. For those families requesting prenatal diagnosis which are not informative for KM.19 and XV-2c, the BanII polymorphism with probe pPT-3 is particularly useful as it is in equilibrium with CF (and the other markers) and will increase the proportion of fully informative families.

In each population studied, the allelic association permits a modification of the risk of a person with a given haplotype being a carrier of the CF mutation for families in which CF has occurred but there is no DNA available from an affected sib. For families with no previous history of CF, the possibility of modifying the risk of being a carrier depends upon the degree of association seen for each linked marker. The disequilibrium in the Danish sample with KM.19 alone is particularly useful in excluding carrier status for the 55% of Danes who are homozygous for the - allele. For other European populations the results are less dramatic, but still permit a useful risk alteration in specific cases requiring genetic counselling.

## E. DNA sequence analysis

The DNA identified according to the present invention can be readily sequenced using conventional procedures. The following shows a part of the DNA from around the HhaI polymorphism of pCS.7. The polymorphism occurs at the point boxed, where the base pair GC provides HhaI site, while the alternative of AT eliminates the site.

```
CCCTGCATAG ACGCGGCACG TCCAAATTTA CAAGTGCTAG CTCTTCATCC CAGCTTCAGG
GGGACGTATC TGCGCCGTGC AGGTTTAAAT GTTCACGATC GAGAAGTAGG GTCGAAGTCC


GAGAGAAGCG AAGCAATGAG TTGAGAATCA TCTCTGGATT CTTGTATCCC ATGCATAGTA
CTCTCTTCGC TTCGTTACTC AACTCTTAGT AGAGACCTAA GAACATAGGG TACGTATCAT


ATCTCCTTAT CCCCTGGCCC CCTTCCTCGT TTCCTCACAT TGCACGCTCA GGGACTTGTT
TAGAGGAATA GGGGACCGGG GGAAGGAGCA AAGGAGTGTA ACGTGCGAGT CCCTGAACAA


TGCCAGCGGA TGGCCTCGGC AATCCGGAAC GCGCGCTCCG AGAGCCCACG GATGCTCTTT
ACGGTCGCCT ACCGGAGCCG TTAGGCCTTG CGCGCGAGGC TCTCGGGTGC CTACGAGAAA


GGCCTGGAGC TTCCCTAAAG GTTCCTGTAT TCGCGTGTGC TCGTAACCAT GCAGCGATGT
CCGGACCTCG AAGGGATTTC CAAGGACATA AGCGCACACG AGCATTGGTA CGTCGCTACA


TCCCCCTTCC CCGCCTCACC TCATCCCCAG ACATCTCTTG CCATCATTTC ATGCACCCGT
AGGGGGAAGG GGCGGAGTGG AGTAGGGGTC TGTAGAGAAC GGTAGTAAAG TACGTGGGCA


GTCTAAAACC CCGCGTTTCT CCCCACCCCC GCCAGGCGCA GCACCCCCTC CCTCGGCTGC
CAGATTTTGG GGCGCAAAGA GGGGTGGGGG CGGTCCGCGT CGTGGGGGAG GGAGCCGACG


GCCCGGAGGG GAGCAGAGCG GACGGGGCGC GCGGGAGGCG CGCAGAGCTT TCGGGCTGCA
CGGGCCTCCC CTCGTCTCGC CTGCCCCGCG CGCCCTCCGC GCGTCTCGAA AGCCCGACGT


GGCGCTCGCT GCGCTGGGGA ATTGGGCTGT GGGCGAGGCG GTCCGGGCTG GCCTTTATCG
CCGCGAGCGA CGCGACCCCT TAACCCGACA CCCGCTCCGC CAGGCCCGAC CGGAAATAGC


CTCGCTGGGC CCATCGTTTG AAACTTTATC AGCGAGTCTC GCCACTCGTC GCAGAGCCAG
GAGCGACCCG GGTAGCAAAC TTTGAAATAG TCGCTCAGAG CGGTGAGCAG CGTCTCGGTC


CGGGGGGCGG GGGCGCGGCG AGGCGGCGGC CGTGACGAGG CGCTCCCGGA GCTGAGCGCT
GCCCCCCGCC CCCGCGCCGC TCCGCCGCCG GCACTGCTCC GCGAGGGCCT CGACTCGCGA


TCTGCTCTGG GCACGCATGG CGCCCGCACA CGGAGTCTGA CCTGATGCAG ACGCAAGGGG
AGACGAGACC CGTGCGTACC GCGGGCGTGT GCCTCAGACT GGACTACGTC TGCGTTCCCC

     start
GTTAATATGA ACGCCCCTCT CGGTGGAATC TGGCTCTGGC TCCCTCTGCT CTTGACCTGG
CAATTATACT TGCGGGGAGA GCCACCTTAG ACCGAGACCG AGGGAGACGA GAACTGGACC
```

Also indicated is the putative ATG start codon for a coding sequence extending downstream of the HTF island. The coding sequence is interrupted by introns in the genomic DNA, but transcription of the DNA in a transformed host produced mRNA from which a cDNA library was prepared by reverse transcription. It was from this library that the probe pPT-3 was obtained.

Synthetic DNA based on the above sequence can also be used for example as a probe to isolate pCS.7 from a DNA bank or library, or to provide primer sequences for use in PCR based analysis. Likewise, DNA from other regions of the contigs can be sequenced and used in similar procedures.

## F. A simple non-invasive method to obtain DNA for gene analysis

### F.1 Background

Genetic analysis of large numbers of individuals using molecular methods is often limited by the availability of DNA. At present blood leukocytes are the major source of DNA used for gene analysis, yielding approximately 30μg DNA/ml whole blood from a normal person. However, collecting blood is both expensive and time consuming, requiring trained staff and sterile disposable syringes and needles. A high level of informed consent is necessary, and dealing with young children can be difficult.

Particular problems are posed where large numbers of samples are collected, such as schools and colleges, because of legitimate concern about AIDS and hepatitis transmission via needle stick injuries. In third world countries these problems can be insurmountable.

A new technique for gene amplification known as polymerase chain reaction (PCR) has recently been developed that allows the specific amplification of a region of DNA (Scharf S., et al. (1986), Science 233:1076-1078). This procedure obviates the need for highly pure DNA on which to perform gene analysis. The intrinsic sequence specificity of the amplification allows the desired gene to be amplified from a heterogeneous mixture of human DNA, contaminating sequences and partially degraded DNA.

We have investigated alternatives to blood leukocytes as a primary source of DNA on which to perform the polymerase chain reaction. Genomic DNA was isolated from buccal epithelial cells obtained from a saline mouth wash, and also from single hair follicles. Using PCR we amplified a 330 base pair fragment of DNA contained within pCS.7. Subsequent restriction enzyme digestion of the PCR products allowed rapid and accurate CS-7 haplotype determination.

### F.2. Experimental

Subjects rinsed out their mouths with 15mls 0.9% saline for approximately ten seconds. Buccal epithelial cells were pelleted by centrifugation at 2,000 rpm for 10 mins. The cell pellet was resuspended in 1ml 1% SDS and incubated for 1-2 h at 55°. Alternatively, 1-4 hair roots were freshly taken, and incubated for 1-2 h at 55° in 500μl of STE (100 mM NaCl, 10mM Tris, 1mMEDTA). One phenol/chloroform extraction was performed and the DNA was precipitated with ethanol. Alternatively, the pelleted cells were dissolved in 0.5 mls water and boiled for 10 minutes. Cellular debris was pelleted by centrifugation for 30 seconds and 50 μl of supernatant was used directly for PCR amplification (Kogan, S., et al. (1987), New Eng. J. Med. 316:985-990. Saiki, R., et al. (1988) Science 239:487-491. Saiki, R., et al (1987), Science 230:1350-1354. Embury, S., et al. (1987) New Eng. J. Med. 316:656-61. DiLella, A., et al. (1988), Lancet ii:497-499. This procedure not only denatures the DNA but also destroys any pathogens in the saliva.

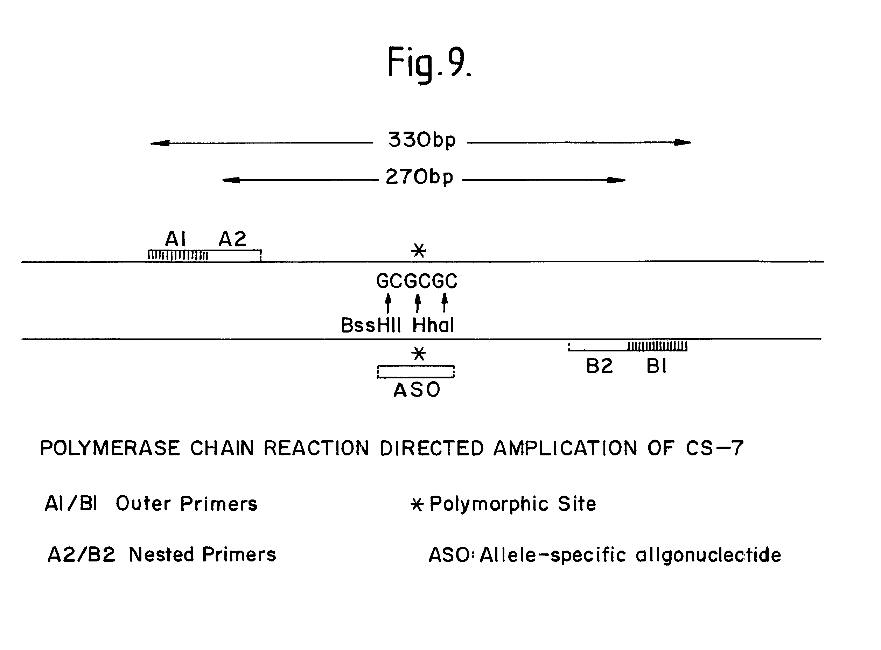

The polymerase chain reaction was used to amplify the polymorphic region of CS-7 using two pairs of primers (30bp long) that flank a 330bp fragment of DNA (Fig. 9). The allele-specific oligonucleotide is 17 bases long and spans the polymorphic site. This site is recognized directly by HhaI digestion and indirectly by BssHII digestion. The 330bp product was obtained using outer primers A1/B1, the 270bp product using A2/B2 nested primers. The procedure was carried out in a 1 x Taq buffer (67mM Tris-Cl pH8.8, 16.6mM ammonium sulphate, 10mM β-mercaptoethanol, 6.7μM EDTA, 6.7mM magnesium chloride, 1.5mM dNTPs and 100pM of each oligonucleotide primer) on the total yield of DNA from buccal epithelium or hair follicles from each sample, and 1μg of control genomic DNA from leukocytes. Prior to the first elongation the reaction mixture was boiled for 10 minutes, cooled to room temperature and 1μl (4μ/μl) Taq DNA polymerase (Anglian Biotech) added.

Extension was at 68° for 4 min. Subsequent cycles were as follows: 93°C 2 mins; room temperature, 1 min; 68°C, 4 mins. One μl of Taq polymerase was added every 5th cycle. After 10 cycles 1/10th of the reaction mixture was removed, to which was added 100 pM of each of the nested primers and PCR was continued for a further 20 cycles under identical conditions. The amplified DNA fragment was resolved by agarose gel electrophoresis, and confirmation that the correct sequence had been amplified was established by low stringency hybridization to an allele specific oligonucleotide probe (ASO).

### F.3 Results

We were able to amplify selectively the polymorphic region of CS-7 using DNA from blood, buccal epithelia and hair follicles. Control DNA from a family already haplotyped for CS-7 using Southern blotting was used to confirm that correct haplotyping by digestion of PCR products could be achieved. PCR products were digested with HhaI or BssHII, and resolved on either agarose or acrylamide gels. Hybridisation with an oligonucleotide probe specific for the presence of the polymorphic site confirmed that the correct sequence had been amplified. We used both nested and un-nested sets of primers, and, although we find that specificity is increased using nested primers the advent of thermostable Taq DNA polymerase which permits

0 288 299

amplification to occur at the more stringent extension temperature of 70° allows greater specificity with a single primer pair.

We obtained satisfactory results after digestion of amplified DNA from buccal cells from mouth wash. We found hair follicles more difficult to handle than buccal epithelium; the overall yield of total genomic DNA is only bout 200ng per root. One 15ml mouth wash produces a far greater yield of total genomic DNA, approximately 2 to 5 µg, sufficient for numerous PCR reactions. Using hair follicles as a source of DNA will have greater application to forensic studies where other cells are not readily available. We have therefore shown that rapid genetic analysis can now be achieved using PCR on DNA obtained from buccal cells and hair follicles. The use of cells from a simple mouthwash, followed by boiling in water and immediate amplification with thermostable DNA polymerase, should be a simple and useful procedure in carrier testing the population for single gene disorders for which the mutation is known, and will also aid prenatal diagnosis of diseases such as cystic fibrosis where closely linked markers are available.

Simultaneous amplification of two unrelated sequences (e.g. sickle cell anaemia and cystic fibrosis) enables multiple diagnosis on a single sample. In cases where haplotype analysis can be performed by restriction enzyme digestion alone, it is possible to eliminate the use of radio-active isotopes, increasing safety and reducing costs. PCR lends itself well to automation, and using thermostable DNA polymerase it should be possible to eliminate the enzyme addition steps currently performed during the amplification procedure.

G. Conclusions

The information provided herein, particularly the pKM.19 probe, enables CF screening to be carried out far more effectively than hitherto, and promises further important developments.

The probe can be used in conjunction with haplotyping, or carrier exclusion as indicated earlier, and would enable about two-thirds of the population to be excluded from significant risk.

Because the highest levels of linkage disequilibrium are found towards the 5' end of the genomic DNA represented by the contigs, probe pKM.19 is preferred for use. However, should it prove uninformative for CF in a case under investigation, probe pCS.7, and if necessary probe pXV-2c can be tried. It should not be necessary in practice to have to resort to probe pPT-3, although the latter could still be useful for prenatal diagnosis in a family, as opposed to the population at large.

In prenatal diagnosis, the fetal DNA from a high risk couple can be sampled, e.g. by CVS, and screened using the probe, and a termination of the pregnancy offered if appropriate.

Other probes can doubtless be obtained from within the contigs described. The foregoing description exemplifies particularly four restriction polymorphisms, at least three of which can be used effectively and easily for diagnosis. However continued analysis of the contigs should reveal other polymorphisms which may provide probes at least as good as some or all of those exemplified above. Apart from high linkage disequilibrium, associated with polymorphisms more towards the 5' end of the contigs, probes are preferred which avoid multiple repeat sequences, as these tend to give less clear hybridisation in Southern blotting, which at present is the more standard method of using the probes. This may be less of a problem with other techniques, such as the more recently introduced direct gene analysis using DNA amplification by polymerase chain reaction (PCR).

The genomic DNA to be tested can be taken from blood, chorion, buccal epithelial cells, hair root, blastomere, sperm, ovum or any other human tissue.

The probes can be obtained using the procedures indicated herein. However, it will be apparent to those skilled in the art that the information given herein enables a known probe to be obtained in far simpler ways. For example, force cloning of human DNA can be used. This involves cutting total human DNA simultaneously with a pair of rare cutter restriction enzymes, for example selected from those of the HTF shown in Fig. 3, separating the fragments by size and cloning those of the appropriate size, and then screening by restriction mapping to find a clone with the restriction pattern corresponding to pCS.7. Alternatively, using a DNA sequence already determined for part of the genomic DNA, an oligonucleotide probe can be prepared and used to screen the restriction fragment colonies.

Claims

1. Human genomic DNA from chromosome 7q22-31, of about 80 kb, as represented in Figs. 2 and 6 hereof, and comprising a NotI/HindIII sequence of about 2.0 kb identifiable by the endonuclease restriction map shown in Fig. 3 hereof.

2. A DNA hybridisation probe having a sequence corresponding to a fragment of the genomic DNA of Claim 1, and comprising a restriction fragment length polymorphism (RFLP) in linkage disequilibrium with cystic fibrosis (CF).

3. A DNA probe according to claim 2 wherein the RFLP is selected from the PstI, HhaI, TaqI and BanII polymorphisms as represented in Fig. 6 hereof.

4. A DNA probe according to claim 2 wherein the RFLP is selected from the PstI, HhaI and TaqI polymorphisms as represented in Fig. 6 hereof.

14

5. A DNA probe according to claim 2 wherein the RFLP is the PstI polymorphism as represented in Fig. 6 hereof.

6. A method of CF risk diagnosis which involves hybridising a sample of genomic DNA from a human subject with one or more DNA probes each having a RFLP in linkage disequilibrium with CF; characterised in the use of a probe according to any one of claims 2 to 5.

7. A method according to claim 6 wherein the subject individual is not known to be from a family in which CF has occurred, whereby the subject is diagnosed as to the probable risk of being a genetic carrier for CF.

8. A method according to claim 6 wherein the subject individual is from a family in which CF is known to occur, and the family is informative for the polymorphism of the probe, whereby the risk of the subject being a genetic carrier for CF is diagnosed.

9. A method according to claim 6 wherein the subject individual is a fetus in a family in which CF is known to occur or where one or both parents are known to be, or to have a high risk of being, carriers for CF, whereby the fetus is diagnosed as to whether it is homozygous for the probe polymorphism.

10. A method according to any one of claims 6 to 9 wherein more than one probe of any one of claims 2 to 5 is used.

11. A method according to any one of claims 6 to 10 in which the hybridisation is carried out by the method of Southern blotting.

12. A method according to any one of claims 6 to 10 in which the hybridisation is carried out on genomic DNA which has been subjected to amplification by the method of polymerase chain reaction (PCR).

13. A method according to claim 12 wherein the genomic DNA is obtained either from a sample of blood, chorion, buccal epithelial cells, hair roots, blastomere, sperm, ovum or any other human tissue.

14. A diagnostic kit for carrying out a method of CF risk diagnosis which involves hybridising a sample of genomic DNA from a human subject with one or more DNA probes each having a RFLP in linkage disequilibrium with CF, wherein the kit includes a DNA probe and one or more other components required for carrying out the method; characterised in that said DNA probe is a probe of any one of claims 2 to 5.

### Claims for the following Contracting State: ES

1. A process which comprises the isolation of human genomic DNA from chromosome 7q22-31, of about 80 kb, as represented in Figs. 2 and 6 hereof, and comprising a NotI/HindIII sequence of about 2.0 kb identifiable by the endonuclease restriction map shown in Fig. 3 hereof.

2. A process which comprises the preparation of a DNA hybridisation probe having a sequence corresponding to a fragment of the human genomic DNA from within the isolate of claim 1 and comprising a restriction fragment length polymorphism (RFLP) in linkage disequilibrium with cystic fibrosis (CF)

3. A process according to claim 2 wherein the RFLP is selected from the PstI, HhaI, TaqI and BanII polymorphisms as represented in Fig. 6 hereof.

4. A process according to claim 2 wherein the RFLP is selected from the PstI, HhaI and TaqI polymorphisms as represented in Fig. 6 hereof.

5. A process according to claim 2 wherein the RFLP is the PstI polymorphism as represented in Fig. 6 hereof.

6. A method of CF risk diagnosis which involves hybridising a sample of genomic DNA from a human subject with one or more DNA probes each having a RFLP in linkage disequilibrium with CF; characterised in the use of a probe according to any one of claims 2 to 5.

7. A method according to claim 6 wherein the subject individual is not known to be from a family in which CF has occurred, whereby the subject is diagnosed as to the probable risk of being a genetic carrier for CF.

8. A method according to claim 6 wherein the subject individual is from a family in which CF is known to occur, and the family is informative for the polymorphism of the probe, whereby the risk of the subject being a genetic carrier for CF is diagnosed.

9. A method according to claim 6 wherein the subject individual is a fetus in a family in which CF is known to occur or where one or both parents are known to be, or to have a high risk of being, carriers for CF, whereby the fetus is diagnosed as to whether it is homozygous for the probe polymorphism.

10. A method according to any one of claims 6 to 9 wherein more than one probe of any one of claims 2 to 5 is used.

11. A method according to any one of claims 6 to 10 in which the hybridisation is carried out by the method of Southern blotting.

12. A method according to any one of claims 6 to 10 in which the hybridisation is carried out on genomic DNA which has been subjected to amplification by the method of polymerase chain reaction (PCR).

13. A method according to claim 12 wherein the genomic DNA is obtained either from a sample of blood, chorion, buccal epithelial cells, hair roots, blastomere, sperm, ovum or any other human tissue.

14. A diagnostic kit for carrying out a method of CF risk diagnosis which involves hybridising a sample of genomic DNA from a human subject with one or more DNA probes each having a RFLP in linkage disequilibrium with CF, wherein the kit includes a DNA probe and one or more other components required for carrying out the method; characterised in that said DNA probe is a probe of any one of claims 2 to 5.

# Fig.1.

CIId CELL LINE (= IMb HUMAN DNA)

HIGH MOLECULAR WEIGHT DNA

HindIII PARTIAL DIGESTION > 100kb SIZE CUT

RECUT XmaIII

SIZE SEPARATION 40-50 kb FRAGMENTS

LIGATE TO LORIST-6 HindIII/NotI DIGESTED

PLATE ON SUP⁻ HOST

FIVE HUMAN COSMIDS

COS INSERT COS

# Fig.2.

-35  -30  -25  -20  -15  -10  -5   0   5   10   15   20   25   30   35   40   45   50kb

pXV-2a

N                    cNX.2                    H

H           cNX.4            N

H                    cLX.261                    H

H        cHI47/cLX.148        H

H           cH.188           H

H           cH3           H

Fig.3.

0288299

# Fig.4.

kb

-2.1

-1.4

-1.2

# Fig.5.

kb

-0.57

-0.47

-0.22

# Fig.6.

5 kb

5'_____3'

cNX.2

cNX.4

cLX.261

HTF

PstI
(pkM.19)

HhaI
(pCS.7)

TaqI
(pXV-2c)

BanII
(pPT-3)

# Fig.7.

7.8
6.6

# Fig.8.

0.7

0.5

# Fig.9.

POLYMERASE CHAIN REACTION DIRECTED AMPLICATION OF CS−7

AI/BI  Outer Primers          * Polymorphic Site

A2/B2 Nested Primers          ASO: Allele-specific allgonuclectide